# EUROPEAN PATENT SPECIFICATION

(11) **EP 2 345 741 B1**
(45) Date of publication and mention of the grant of the patent: **10.09.2014**
(21) Application number: 09819288.3
(22) Date of filing: 06.10.2009
(51) Int. Cl.: C12Q 1/68, C12N 15/09, C12Q 1/44, C12Q 1/48, G01N 33/53

(54) **METHOD FOR MEASURING SURVIVIN mRNA**
VERFAHREN ZUR SURVIVIN-MRNA-MESSUNG
PROCÉDÉ POUR MESURER L'ARNm DE LA SURVIVINE

(30) Priority: 06.10.2008 JP 2008259366
(43) Date of publication of application: 20.07.2011
(73) Proprietor: Tosoh Corporation, Yamaguchi 746-8501 (JP)
(72) Inventor: OMOTO, Daisuke, Ayase-shi Kanagawa 252-1123 (JP); SAITO, Juichi, Ayase-shi Kanagawa 252-1123 (JP); OONAKA, Satoru, Ayase-shi Kanagawa 252-1123 (JP); HAYASHI, Toshinori, Ayase-shi Kanagawa 252-1123 (JP)
(74) Representative: Kügele, Bernhard
(86) International application number: PCT/JP2009/067687
(87) International publication number: WO 2010/041755

(56) References cited:
- EP-A2- 1 780 289
- WO-A2-02/26968
- WO-A2-2006/050732
- WO-A2-2008/098248
- JP-A- 2000 014 400
- KAMIHIRA, S. ET AL.: 'Aberrant expression of caspase cascade regulatory genes in adult T-cell leukaemia: survivin is an important determinant for prognosis' BR. J. HAEMATOL. vol. 114, 2001, pages 63 - 69, XP008145678
- WANG Z. ET AL.: 'Expression of survivin mRNA in peritoneal lavage fluid from patients with gastric carcinoma' CHIN. MED. J. vol. 117, no. 8, 2004, pages 1210 - 1217, XP008145690
- WEIKERT S. ET AL.: 'Quantitative analysis of survivin mRNA expression in urine and tumor tissue of bladder cancer patients and its potential relevance for disease detection and prognosis' INT. J. CANCER vol. 116, 2005, pages 100 - 104, XP008145686
- ISHIGURO T. ET AL.: 'Intercalation activating fluorescence DNA probe and its application to homogeneous quantification of a target sequence by isothermal sequence amplification in a closed vessel' ANAL. BIOCHEM. vol. 314, 2003, pages 77 - 86, XP001165733
- TONG, Q. ET AL.: 'Selection of optimal antisense accessible sites of survivin and its application in treatment of gastric cancer' WORLD J. GASTROENTEROL. vol. 11, no. 5, 2005, pages 634 - 640, XP008145682

### SEQUENCE LISTING

<110> TOSOH CORPORATION
<120> Method for Measuring Survivin mRNA
<130> W743-PCT
<150> JP 2008-259366
   <151> 2008-10-06
<160> 34
<210> 1
   <211> 43
   <212> DNA
   <213> Artificial Sequence
<220>
   <223> SurF1
<400> 1
   ccctttctca aggaccaccg catctctaca ttcaagaact ggc 43
<210> 2
   <211> 136
   <212> DNA
   <213> Artificial Sequence
<220>
   <223> SurF2
<400> 2
   gcctgcaccc cggagcggat ggccgaggct ggcttcatcc actgccccac tgagaacgag 60
   ccagacttgg cccagtgttt cttctgcttc aaggagctgg aaggctggga gccagatgac 120
   gaccccatag aggaac 136
<210> 3
   <211> 93
   <212> DNA
   <213> Artificial Sequence
<220>
   <223> SurR1
<400> 3
   cggacgaatg ctttttatgt tcctctatgg ggtcgtcatc tggctcccag ccttccagct 60
   ccttgaagca gaagaaacac tgggccaagt ctg 93
<210> 4
   <211> 137
   <212> DNA
   <213> Artificial Sequence
<220>
   <223> SurR2
<400> 4
   ttttgttctt ggctctttct ctgtccagtt tcaaaaattc accaagggtt aattcttcaa 60
   actgcttctt gacagaaagg aaagcgcaac cggacgaatg ctttttatgt tcctctatgg 120
   ggtcgtcatc tggctcc 137
<210> 5
   <211> 22
   <212> DNA
   <213> Artificial Sequence
<220>
   <223> SurS1
<400> 5
   gagaaagggc tgccaggcag gg 22
<210> 6
   <211> 24
   <212> DNA
   <213> Artificial Sequence
<220>
   <223> SurS2
<400> 6
   tagagatgcg gtggtggttg agaa 24
<210> 7
   <211> 23
   <212> DNA
   <213> Artificial Sequence
<220>
   <223> SurS3
<400> 7
   ggtgcaggcg cagccctcca aga 23
<210> 8
   <211> 22
   <212> DNA
   <213> Artificial Sequence
<220>
   <223> SurS4
<400> 8
   gcctcggcca tccgctccgg gg 22
<210> 9
   <211> 20
   <212> DNA
   <213> Artificial Sequence
<220>
   <223> SurS5
<400> 9
   ctggctcgtt ctcagtgggg 20
<210> 10
   <211> 19
   <212> DNA
   <213> Artificial Sequence
<220>
   <223> SurS6
<400> 10
   ctggctccca gccttccag 19
<210> 11
   <211> 20
   <212> DNA
   <213> Artificial Sequence
<220>
   <223> SurS7
<400> 11
   gtcatctggc tcccagcctt 20
<210> 12
   <211> 21
   <212> DNA
   <213> Artificial Sequence
<220>
   <223> SurF3
<400> 12
   ccctttctca aggaccaccg c 21
<210> 13
   <211> 24
   <212> DNA
   <213> Artificial Sequence
<220>
   <223> SurF4
<400> 13
   gcatctctac attcaagaac tggc 24
<210> 14
   <211> 22
   <212> DNA
   <213> Artificial Sequence
<220>
   <223> SurF5
<400> 14
   gcctgcaccc cggagcggat gg 22
<210> 15
   <211> 20
   <212> DNA
   <213> Artificial Sequence
<220>
   <223> SurF6
<400> 15
   ggccgaggct ggcttcatcc 20
<210> 16
   <211> 20
   <212> DNA
   <213> Artificial Sequence
<220>
   <223> SurF7
<400> 16
   acgagccaga cttggcccag 20
<210> 17
   <211> 20
   <212> DNA
   <213> Artificial Sequence
<220>
   <223> SurF8
<400> 17
   gggagccaga tgacgacccc 20
<210> 18
   <211> 25
   <212> DNA
   <213> Artificial Sequence
<220>
   <223> SurF9
<400> 18
   ccagatgacg accccataga ggaac 25
<210> 19
   <211> 23
   <212> DNA
   <213> Artificial Sequence
<220>
   <223> SurR3
<400> 19
   gaagaaacac tgggccaagt ctg 23
<210> 20
   <211> 21
   <212> DNA
   <213> Artificial Sequence
<220>
   <223> SurR4
<400> 20
   atggggtcgt catctggctc c 21
<210> 21
   <211> 22
   <212> DNA
   <213> Artificial Sequence
<220>
   <223> SurR5
<400> 21
   tttatgttcc tctatggggt cg 22
<210> 22
   <211> 25
   <212> DNA
   <213> Artificial Sequence
<220>
   <223> SurR6
<400> 22
   cggacgaatg ctttttatgt tcctc 25
<210> 23
   <211> 25
   <212> DNA
   <213> Artificial Sequence
<220>
   <223> SurR7
<400> 23
   ttcaccaagg gttaattctt caaac 25
<210> 24
   <211> 25
   <212> DNA
   <213> Artificial Sequence
<220>
   <223> SurR8
<400> 24
   tccagtttca aaaattcacc aaggg 25
<210> 25
   <211> 26
   <212> DNA
   <213> Artificial Sequence
<220>
   <223> SurR9
<400> 25
   ttttgttctt ggctctttct ctgtcc 26
<210> 26
   <211> 20
   <212> DNA
   <213> Artificial Sequence
<220>
   <223> SurR10
<400> 26
   ggcagtggat gaagccagcc 20
<210> 27
   <211> 21
   <212> DNA
   <213> Artificial Sequence
<220>
   <223> SurR11
<400> 27
   tcgttctcag tggggcagtg g 21
<210> 28
   <211> 20
   <212> DNA
   <213> Artificial Sequence
<220>
   <223> SurINAF1
<400> 28
   caggcgcagc cctccaagaa 20
<210> 29
   <211> 21
   <212> DNA
   <213> Artificial Sequence
<220>
   <223> SurINAF2
<400> 29
   ccagctcctt gaagcagaag a 21
<210> 30
   <211> 20
   <212> DNA
   <213> Artificial Sequence
<220>
   <223> SurINAF3
<400> 30
   aaccggacga atgcttttta 20
<210> 31
   <211> 22
   <212> DNA
   <213> Artificial Sequence
<220>
   <223> SurINAF4
<400> 31
   gcttcttgac agaaaggaaa gc 22
<210> 32
   <211> 21
   <212> DNA
   <213> Artificial Sequence
<220>
   <223> SurINAF5
<400> 32
   cggccatccg ctccggggtg c 21
<210> 33
   <211> 20
   <212> DNA
   <213> Artificial Sequence
<220>
   <223> SurINAF6
<400> 33
   gctcgttctc agtggggcag 20
<210> 34
   <211> 28
   <212> DNA
   <213> Artificial Sequence
<220>
   <223> T7 promoter
<400> 34
   aattctaata cgactcacta tagggaga 28

## Description

### TECHNICAL FIELD

The present invention relates to a simple and rapid method of measuring survivin mRNA using a nucleic acid amplification method. More precisely, the present invention provides an oligonucleotide suitable for amplification and detection of survivin mRNA at a constant temperature (from 40 to 50°C, and preferably 43°C). The present invention is useful for research, diagnosis and treatment in the fields of molecular biology, biochemistry, medicine and the like.

### BACKGROUND ART

Apoptosis is a phenomenon by which cells are actively guided to their own death, and is also referred to as programmed cell death. In multicellular organisms like humans, cells disadvantageous for maintaining life are known to be constantly produced, for example by infection of somatic cells with viruses, canceration and the like. Apoptosis is a system by which such cells are removed. Apoptosis also plays other roles essential for life during the course of development. Apoptosis functions via a mechanism that is common to organisms ranging from insects to humans, and within that mechanism, caspase is known to be a key enzyme. Substances that inhibit apoptosis by inhibiting caspase are inhibitors of apoptosis (IAP) proteins.

Typical IAP proteins have a structure consisting of a BIR domain that binds with protein, and an RING domain that is a type of Zn fingered domain. Recently, a protein known as survivin has been identified as a member of inhibitors of apoptosis (IAP) gene family. Instead of a single BIR domain and RING finger, survivin has a coiled coil region, and is structurally unique in comparison with known IAP family members.

One area of clinical importance of survivin is that, differing from another apoptosis regulatory factor, BcI-2, and other IAP family members, although hardly any expression of survivin is detected in normal human tissue, expression has been reported to be significantly increased in common human cancer tissue (such as that of the lungs, pancreas, colon, urinary bladder, thoracic region, prostate gland, stomach and liver) and in non-Hodgkin's lymphoma and leukemia. This indicates that survivin can be an extremely superior marker gene. Consequently, measurement of survivin mRNA is considered to be capable of being applied to a wide range of cancer therapy applications, such as monitoring of apoptosis control, early cancer diagnosis, diagnosis of cancer metastasis and monitoring of the efficacy of cancer therapy.

Although cancer is typically diagnosed pathologically, this requires a high degree of skill on the part of the pathologist, and the potential for overlooking cancer cells has been indicated depending on the specimen used. In recent years, gene testing using nucleic acid amplification has been proposed or is being used practically at some facilities for the purpose of reducing this potential for overlooking cancer cells or enabling uniform diagnoses between hospitals and other health care facilities. Although gene testing using nucleic acid amplification is known to be highly sensitive, the type of cancer marker gene used has a considerable effect on the specificity of that testing in terms of carrying out nucleic acid amplification. As was previously described, survivin has been observed to be highly expressed in a wide range of cancers, lymphomas and leukemias, while on the other hand, has been observed to hardly be expressed at all in normal cells. Thus, survivin has the characteristic of being a superior cancer marker in nucleic acid amplification. Namely, malignant diseases such as cancer, lymphoma or leukemia can be discovered by investigating for the presence or absence of expression of survivin mRNA in various tissues.

Cancer is known to undergo metastasis from its primary site of origin to a remote location in the body. Metastasis is currently the leading cause of death among cancer patients, and diagnosis of metastasis is extremely important in the same manner as diagnosis of the primary site. Cancer cells are released from the primary site into various body fluids such as blood, urine and lymph. When cancer cells are released into the blood or lymph in particular, they circulate throughout the body as a result of being carried in that flow, thereby causing metastasis at numerous sites in the body. Since cancer cells that have been released from the primary site in this manner are typically in small numbers, a detection method having high sensitivity is required to detect these cancer cells. Since detection methods using nucleic acid amplification typically enable highly sensitive detection, they are suitable for detection of these cancer cells. Namely, detection of survivin mRNA using nucleic acid amplification can be said to be a particularly useful detection method for diagnosis metastasis of a wide range of cancers.

As an example thereof, in gastrointestinal cancers such as gastric cancer or colon cancer, the abdominal cavity is lavaged with physiological saline to diagnosis peritoneal metastasis, and the lavaged fluid is examined for the presence of cancer cells. Although cancer cells are exfoliated into the peritoneum when cancers such as gastric cancer progress, a diagnosis of lavage cytodiagnosis positive is made when cancer cells are confirmed to be present in lavaged fluid, and this indicates that the cancer cells cannot be completely removed surgically. Although this examination is normally performed pathologically using a microscope, it is accompanied by difficulty in visually confirming all cells without overlooking cancer cells. In addition, even if cytodiagnosis by pathological examination is negative, numerous cases have been known to present with peritoneal metastasis, and this is thought to be the result of having overlooked a very small number of metastatic cells. Accordingly, a method is sought that enables cancer cells present in lavaged fluid to be detected with high sensitivity. Detection of survivin mRNA using nucleic acid amplification can be considered to enable highly sensitive diagnosis of peritoneal metastasis in all types of gastrointestinal cancers such as gastric cancer or colon cancer.

As another example thereof, cancer cells are known to exfoliate into urine in urinary bladder cancer. In cases in which bladder cancer is suspected, although examinations are typically performed by cytodiagnosis and cystoscopy, the former examination has been indicated as having the risk of overlooking cancer cells as previously described, while the latter has the shortcoming of placing a considerable burden on the patient since the examination involves an invasive procedure.
Consequently, measurement of survivin mRNA by using cells present in urine for the specimen can be said to be an examination method that compensates for the shortcomings of cytodiagnosis and cystoscopy. In addition, although examinations using blood for the specimen are applicable when focusing on the aspect of being lowly invasive, detection of survivin mRNA using nucleic acid amplification enables cancer cells to be detected with high sensitivity in a wide range of cancers even though blood is used for the specimen.

In addition, as another example thereof, cancer cells may also exfoliate into pleural fluid in lung cancer or peritoneal fluid in liver cancer. Exfoliation of cancer cells into body fluids in this manner typically serves as an indicator of the degree of progression of the cancer. The presence of cancer cells in pleural fluid or peritoneal fluid in this manner can be detected with high sensitivity by detecting survivin mRNA using nucleic acid amplification.

Moreover, as still another example thereof, measurement of survivin mRNA can also be applied to diagnosis of lymph node metastasis during surgery. At present, the importance of diagnosis sentinel lymph node metastasis in breast cancer is widely recognized, and application is also being studied in gastrointestinal cancers including gastric cancer. In this manner, based on its expression characteristics, survivin is considered to be an extremely useful cancer marker gene, and therefore has a wide application range and a high degree of usefulness.

In general, during detection of cancer marker gene mRNA using nucleic acid amplification, reverse transcription-polymerase chain reaction (RT-PCR) is used for nucleic acid amplification, and it has also been reported to have been applied to survivin mRNA (refer to Mertines, A., et al., American Journal of Pathology, 164, 501-510 (2004) (Non-Patent Document 1); Zhen-ning, W., et al., Chinese Medical Journal, 117, 1210-1217 (2004) (Non-Patent Document 2); and, Weikert, S. et al., International Journal of Cancer, 116, 100-104 (2005) (Non-Patent Document 3)). Since nucleic acid amplification requires two steps indicated below following extraction of RNA from tumor tissue, i.e.,
(a) a step for synthesizing cDNA from the extracted RNA with reverse transcriptase, and
(b) a step for detecting the cDNA by amplifying by PCR,
(and it may be necessary to separately carry out an additional detection step depending on the case), complexity of the procedure and the risk of secondary infection are suggested. In addition, since it normally requires 2 hours or more to carry out the two steps, there have been problems with respect to poor reproducibility attributable to carrying out multiple steps as well as reducing large-volume specimen processing and testing costs. Moreover, since amplification by PCR involves amplification of double-stranded DNA, there is concern over the possibility of amplifying contaminating chromosomal DNA, thereby resulting in the need to completely removal all chromosomal DNA by digesting with DNase and the like in order to precisely analyze mRNA expression. Consequently, this results in greater complexity of the procedure and poorer reproducibility. Moreover, since the PCR method requires the reaction temperature to be rapidly raised and lowered, it serves as an obstacle to labor saving and cost reduction of reaction devices at the time of automation.

On the other hand, examples of other methods used to amplify only RNA at a constant temperature include NASBA (refer to Japanese Patent Publication No. 2650159 (Patent Document 1) and Japanese Patent Publication No. 3152927 (Patent Document 2) and TMA (Japanese Patent Publication No. 3241717 (Patent Document 3)). These RNA amplification methods involve synthesizing double-stranded DNA containing a promoter sequence by use of a primer to a target RNA wherein the primer comprises the promoter sequence, reverse transcriptase and, as necessary, ribonuclease H (RNase H), producing RNA containing a specific base sequence derived from the target RNA by use of RNA polymerase with using the double-stranded DNA as template, and using this RNA to carry out a chain reaction using double-stranded DNA containing a promoter sequence as template. Following RNA amplification, the amplified RNA is detected by electrophoresis or a hybridization method using a nucleic acid probe bound with a detectable label.

Although these RNA amplification methods are suitable for easily measuring RNA since they amplify only RNA at a constant temperature and in a single step, since the hybridization procedure and the like requires a complex procedure, not only are they not suitable for large-volume specimen processing and automation, they also have the shortcomings of poor reproducibility and the potential for secondary contamination by amplified nucleic acids as a result thereof. In addition, it normally takes 90 minutes or more to obtain results for both NASBA and TMA, thus preventing results from being obtained rapidly. Moreover, although the amplification step is carried out at a constant temperature, since the amplification step usually requires preheating (at a temperature of, for example, 65°C), these methods have shortcomings with respect to labor saving and reducing costs of the reaction apparatus.

An example of a method for easily amplifying and measuring RNA is the method of Ishiguro, et al. (refer to Japanese Unexamined Patent Publication No. 2000-14400 (Patent Document 4) and Ishiguro, T. et al., Analytical Biochemistry, 314, 1247-1252 (2003) (Non-Patent Document 4)). This method involves carrying out RNA amplification in the presence of an oligonucleotide probe labeled with an intercalating fluorescent dye and designed so that when it forms a complementary double-strand with the target nucleic acid, the intercalating fluorescent dye moiety undergoes a change in fluorescent properties due to intercalation into the double-stranded moiety, and measuring the change in fluorescent properties, thereby enabling amplification and measurement of RNA to be carried out simultaneously, rapidly and easily at a constant temperature, in a single step and in a closed vessel. In a more specific aspect thereof, this method consists of carrying out the following steps on a specific base sequence that allows an arbitrary RNA to be distinguished from other RNA in the presence of that RNA:
(1) forming a DNA complementary to the specific base sequence by use of a DNA primer complementary to the 3'-end of the specific base sequence, RNA-dependent DNA polymerase (reverse transcriptase) and RNA as a template,
(2) forming a single-stranded DNA by allowing an enzyme having ribonuclease H activity to act on the double-stranded RNA-DNA formed by the reverse transcription reaction of (1) to decompose the RNA,
(3) synthesizing a double-stranded DNA containing an RNA polymerase promoter sequence by use of a DNA primer complementary to the 3'-end of the single-stranded DNA formed in (2) and having the RNA polymerase promoter sequence on the 5'-end thereof, and DNA-dependent DNA polymerase, and
(4) forming a transcription product (the RNA of the specific base sequence) by allowing RNA polymerase to act on the double-stranded DNA formed in (3).

Since the RNA transcription product formed in (4) is an RNA derived from the specific base sequence, it serves as a template in the reaction of (1), binds to the DNA primer used in the reaction of (1), and allows the reactions of (1) to (4) to proceed to cause an RNA amplification chain reaction.

This nucleic acid amplification method is characterized by not requiring the temperature of the reaction solution to be raised and lowered in the manner of PCR, and carrying out reverse transcription of RNA and the subsequent DNA amplification reaction separately. Moreover, the state of amplification can be detected (monitored) simultaneous to amplifying the specific base sequence or sequence complementary to that sequence by also incorporating the oligonucleotide probe labeled with the intercalating fluorescent dye capable of specifically binding to the amplified RNA transcription product present in the reaction solution. Thus, since it is not necessary to carry out hybridization detection and the like separately in the manner of ordinary RNA amplification, the amount of time required for results to be obtained can be shortened considerably.

However, a primer sequence for amplification of survivin mRNA suitable for this nucleic acid amplification method or combinations thereof, or an oligonucleotide probe sequence for detection, are currently not known. This is because, in comparison with nucleic acid amplification methods such as PCR, NASBA or TMA, which require a step for temporarily raising the temperature at the start of the reaction to a temperature higher than the reaction temperature to denature the high-dimensional structure of the target RNA, amplification and detection of mRNA in this nucleic acid amplification method are carried out under constant, comparatively low temperature conditions (from 40°C to 50°C, and preferably 43°C). In other words, typical single-stranded RNA in the manner of mRNA is known to easily form a high-dimensional structure, and under reaction conditions like those of this nucleic acid amplification method, the target mRNA forms a high-dimensional structure, and since this is thought to impair binding of the primer and probe, it is necessary to design an optimum primer and probe in a region that does not have a high-dimensional structure. Although it is possible to calculate and estimate secondary structure from nucleic acid sequences using secondary structure analytical software as an indicator of RNA high-dimensional structure, it is extremely difficult to estimate actual high-dimensional structure from a calculated secondary structure.

In addition, in the case of carrying out nucleic acid amplification at a comparatively low temperature as in this nucleic acid amplification method, non-specific products such as primer dimers form easily in comparison with PCR carried out at a high temperature, and in order to reduce the formation of non-specific products, it is necessary to carefully select the combination of primers used. However, since commonly used primer design techniques are premised on containing a step involving denaturation at a high temperature (PCR), it is difficult to design primers suitable for this nucleic acid amplification method using known primer design techniques. Accordingly, in order to realize highly sensitive measurement of survivin mRNA both rapidly and easily by amplifying and measuring mRNA at a constant temperature as described above, an oligonucleotide and combinations thereof are required that do not demonstrate a decrease in binding efficiency and enable amplification and detection of survivin mRNA even under constant, comparatively low temperature conditions (from 40°C to 50°C, and preferably 43°C) .

WO-A-2008/098248 describes the quantification of intracellular survivin RNA levels using RT-PCR.

EP-A-1 780 289 describes a method for assaying Reg IV mRNA using transcription based RNA amplification.

### DISCLOSURE OF THE INVENTION

An object of the present invention is to provide a method of rapidly measuring survivin mRNA with a procedure carried out at a constant temperature and in a single step on a sample obtained from human cells or tissue and the like.

Extensive studies to solve the aforementioned problems conducted by the inventors of the present invention led to the construction of a method of specifically and rapidly measuring survivin mRNA.

A first invention is a method of measuring survivin mRNA in a sample consisting of the following steps that uses a first primer having a sequence homologous with a portion of a specific base sequence in survivin mRNA, and a second primer having a sequence complementary with a portion of the specific base sequence, and one of either the first primer or the second primer is a primer to which has been added to the 5'-end thereof an RNA polymerase promoter sequence:
(1) a step for synthesizing a cDNA complementary to said specific base sequence by use of an enzyme having RNA-dependent DNA polymerase activity which uses RNA as a template;
(2) a step for decomposing the RNA-DNA double-stranded RNA obtained in the reaction of (1) above by use of an enzyme having ribonuclease H (RNase H) activity (formation of single-stranded DNA);
(3) a step for forming double-stranded DNA having a promoter sequence capable of transcribing the specific base sequence or RNA having a sequence complementary to the specific base sequence by use of an enzyme having DNA-dependent DNA polymerase activity with using the single-stranded DNA as template;
(4) a step for forming an RNA transcription product by use of an enzyme having RNA polymerase activity with using the double-stranded DNA as template;
(5) a step for forming an RNA transcription product in a chain reaction by allowing the RNA transcription product to serve as a template of cDNA synthesis in the reaction of (1); and,
(6) a step for measuring the amount of the RNA transcription product;
wherein the first and second primers are either of the oligonucleotides indicated below :
(i) the first primer is one type of oligonucleotide selected from SEQ ID NO: 12 or 13, and the second primer is one type of oligonucleotide selected from any of SEQ ID NO: 19 to 22; or,
(ii) the first primer is one type of oligonucleotide selected from any of SEQ ID NO: 14 to 18, and the second primer is one type of oligonucleotide selected from any of SEQ ID NO: 20 to 25; and
wherein said method is carried out under constant temperature conditions from 40°C to 50°C.

A second invention is the previously described measurement method of measuring survivin mRNA described in the first invention, characterized in that the step described in (6) above (the step for measuring an amount of RNA transcription product) is carried out by measuring a change in fluorescent properties in the presence of a fluorescent dye-labeled oligonucleotide probe designed to change in its fluorescent properties when it forms a complementary double-strand with the target RNA.

A third invention is the measurement method of measuring survivin mRNA according described in the second invention, characterized in that the fluorescent dye-labeled oligonucleotide probe is an intercalating fluorescent dye-labeled oligonucleotide probe in which an intercalating fluorescent dye is bound through a linker.

A fourth invention is the measurement method of measuring survivin mRNA described in the first to third invention, characterized in that prior to the step described in (1) above (the step for synthesizing cDNA complementary to the specific base sequence by use of an enzyme having RNA-dependent DNA polymerase activity which uses with RNA as a template), a step is carried out for cleaving the RNA at the 5'-end site of the specific base sequence by use of a specific base sequence in survivin mRNA as a template, and using:
(i) a cleaving oligonucleotide having a region that overlaps with the 5'-end site of a region homologous to the first primer in the specific base sequence, and a sequence complementary to an adjacent region on the 5' side from the site, and
(ii) an enzyme having ribonuclease H (RNase H) activity.

A fifth invention is an oligonucleotide combination for specifically amplifying or detecting survivin mRNA, characterized by containing a first primer and a second primer, wherein the first and second primers are either of the oligonucleotides indicates below :
(i) the first primer is one type of oligonucleotide selected from SEQ ID NO: 12 or 13, and the second primer is one type of oligonucleotide selected from any of SEQ ID NO: 19 to 22; or,
(ii) the first primer is one type of oligonucleotide selected from any of SEQ ID NO: 14 to 18, and the second primer is one type of oligonucleotide selected from any of SEQ ID NO: 20 to 25.

A sixth invention is a reagent for measuring survivin mRNA characterized by containing at least the oligonucleotide combination of the fifth invention.

### BRIEF DESCRIPTION OF THE DRAWINGS

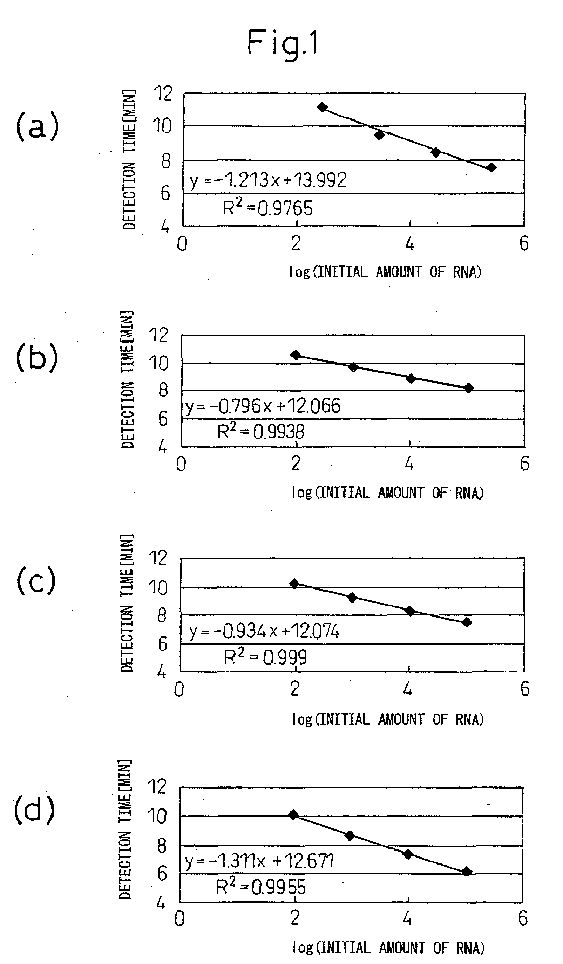

FIG. 1 is a calibration curve of survivin RNA indicating an equation of a linear first order curve and a value of R² determined from each point, wherein (a) indicates oligonucleotide combination [7] of Table 1, (b) indicates combination [15], (c) indicates combination [17] and (d) indicates combination [38], the time when the fluorescence intensity ratio has exceeded 1.2 (detection time, minutes) is plotted on the vertical axis, and the initial amount of standard RNA (number of copies) used for measurement represented as a log is plotted on the horizontal axis.

The following provides a detailed explanation of the present invention.

The sample in the present invention refers to a nucleic acid sample that contains RNA. The present invention measures survivin mRNA contained in human cells or tissue and the like serving as the source of the sample by using human cells, human tissue, body fluid, blood, urine, stool, lymph, nipple aspiration fluid or lavaged fluid from the peritoneal or thoracic cavity as a sample, using the sample prepared based on, for example, the nucleic acid extraction method described in Japanese Unexamined Patent Publication No. H7-59572, and measuring the sample directly.

The specific base sequence in the present invention refers to an RNA or DNA base sequence of the survivin mRNA starting from the 5'-end of a region homologous with the first primer and ending to the 3'-end of a region complementary to the second primer. Namely, in survivin mRNA, the first primer is homologous with at least 15 contiguous bases in the 3' direction from the 5'-end of a specific base sequence, while the second primer is complementary to at least 15 contiguous bases in the 5' direction from the 3'-end of the specific base sequence. Accordingly, in the present invention, an RNA transcription product is amplified that is derived from the specific base sequence. The 5'-end site of a region homologous with the first primer in the present invention refers to a site consisting of a partial sequence containing the 5'-end of the homologous region within the specific base sequence, and the site is a site where a region complementary to the cleaving oligonucleotide and a region homologous with the first primer overlap.

Although promoter sequences are known that are specific to various RNA polymerases at a site where transcription is initiated as a result of binding by RNA polymerase, and there are no particular limitations thereon, a promoter in the present invention is preferably a T7 promoter, SP6 promoter or T3 promoter that is normally used in molecular biology experiments and the like. In addition, an addition sequence involved in transcription efficiency may also be contained in the aforementioned sequence.

The complementary sequence in the present invention refers to a sequence that can hybridize with the target base sequence under highly stringent conditions. An example of highly stringent conditions consists of the composition of the nucleic acid amplification reaction solution described in the examples of the present invention. In addition, a homologous sequence in the present invention refers to a sequence that can hybridize with a completely complementary sequence of a target base sequence under highly stringent conditions. Thus, a complementary or homologous sequence as referred to in the present invention can naturally be set to an arbitrary length and the like provided it is within a range that does not affect specificity or efficiency of hybridization under highly stringent conditions. Moreover, a base sequence may be used in which one or more bases have been substituted, deleted or inserted within a range that does not affect specificity or efficiency of hybridization.

The nucleotide or nucleic acid in the present invention refers to a nucleotide or nucleoside (containing both RNA and DNA) consisting of bases, sugars and intersaccharide bonds that are present in nature, and is a generic term that includes oligomers thereof (oligonucleotides having, for example, about 2 to 100 bases) and polymers (oligonucleotides having, for example, 100 or more bases). A nucleotide or nucleic acid in the present invention includes similarly functioning monomers not present in nature, monomers labeled with a fluorescent molecule or radioisotope and the like, and oligomers or polymers in which they are contained.

The primer in the present invention refers to a nucleotide that hybridizes with a template in a nucleic acid amplification reaction and is required to initiate the nucleic acid amplification reaction, and in the nucleic acid amplification reaction, hybridizes with the template desired to be amplified, and is preferably designed on the base of the template to contain a sequence of the primer itself that is specific to the template so as to obtain a product specific in terms of strand length or sequence by a nucleic acid amplification reaction such as PCR, LAMP, ICAN, NASBA, TMA, 3SR or TRC (refer to Patent Document 4 and Non-Patent Document 4).

In a general manner, the first primer and the second primer for detecting survivin mRNA in the present invention must fulfil either of the requirements indicated below:
(i) the first primer is an oligonucleotide consisting of at least 15 contiguous bases in a sequence homologous to the sequence listed as SEQ ID NO: 1 that is homologous with a portion of survivin mRNA, and the second primer is an oligonucleotide consisting of at least 15 contiguous bases homologous to the sequence listed as SEQ ID NO: 3 that is complementary to a portion of survivin mRNA; or,
(ii) the first primer is an oligonucleotide consisting of at least 15 contiguous bases in a sequence homologous to the sequence listed as in SEQ ID NO: 2 that is homologous with a portion of survivin mRNA, and the second primer is an oligonucleotide consisting of at least 15 contiguous bases in a sequence homologous to the sequence listed as SEQ ID NO: 4 that is complementary to a portion of survivin mRNA.

Furthermore, in the case of (ii), since the 3'-end of SEQ ID NO: 2 and the 3'-end of SEQ ID NO: 4 overlap, it is necessary to design the first primer and the second primer at locations that the 3'-end of the first primer and the 3'-end of the second primer are separated by at least 15 bases.

More particularly, the first primer and the second primer for detecting survivin mRNA in the present invention are either of the oligonucleotides indicated below:
(i) the first primer is one type of oligonucleotide selected from SEQ ID NO: 12 or SEQ ID NO: 13, and the second primer is one type of oligonucleotide selected from any of SEQ ID NO: 19 to 22; or,
(ii) the first primer is one type of oligonucleotide selected from any of SEQ ID NO: 14 to 18, and the second primer is one type of oligonucleotide selected from any of SEQ ID NO: 20 to 25.

Moreover, in one aspect of the present invention, survivin mRNA is cleaved at the 5'-end site of a specific nucleic acid sequence within the RNA prior to serving as a template of cDNA synthesis. As a result of being cleaved at the 5'-end site of a specific nucleic acid sequence, a DNA strand complementary with the promoter sequence of the first primer hybridized to cDNA can be efficiently synthesized following cDNA synthesis by elongating the 3'-end of the cDNA, thereby enabling the formation of a functional double-stranded DNA promoter structure. An example of such a cleavage method consists of cleaving an RNA portion of double-stranded RNA-DNA, formed by adding an oligonucleotide having a sequence complementary to a region that overlaps with a 5'-end site of the specific base sequence within survivin mRNA (partial sequence containing the 5'-end site of the specific base sequence) and is adjacent thereto in the 5'-direction (to be referred to as a cleaving oligonucleotide), with an enzyme having ribonuclease (RNase H) activity. The hydroxyl group on the 3'-end of the cleaving oligonucleotide is preferably suitably modified to prevent an elongation reaction, an example of which is an aminated hydroxyl group.

The cleaving oligonucleotide is preferably an oligonucleotide consisting of a sequence homologous to any of the sequences listed as SEQ ID NO: 5 to 11 that are complementary with a portion of survivin mRNA.

The target RNA in the present invention refers to a sequence among specific base sequences of an RNA transcription product other than a region that is homologous or complementary with the aforementioned primers, and has a sequence capable of complementarily binding with an intercalating fluorescent dye-labeled oligonucleotide probe. Accordingly, the intercalating fluorescent dye-labeled oligonucleotide probe is a sequence that is complementary or homologous with a portion of the specific base sequence in the present invention. The intercalating fluorescent dye-labeled oligonucleotide probe is preferably a probe containing an oligonucleotide consisting of at least 15 bases in a sequence homologous or complementary to SEQ ID NO: 28 to 31 that are complementary with a portion of survivin mRNA.

Examples of combinations of oligonucleotides for detecting survivin mRNA in the present invention include the following:
(i) the cleaving nucleotide is an oligonucleotide consisting of SEQ ID NO: 5, the first primer is an oligonucleotide consisting of SEQ ID NO: 12 (and furthermore, the first primer has a promoter sequence on the 5'-end thereof), the second primer is one type of oligonucleotide selected from SEQ ID NO: 19 to 22, and the intercalating fluorescent dye-labeled oligonucleotide probe is an oligonucleotide consisting of SEQ ID NO: 28 or 29;
(ii) the cleaving nucleotide is an oligonucleotide consisting of SEQ ID NO: 6, the first primer is an oligonucleotide consisting of SEQ ID NO: 13 (and furthermore, the first primer has a promoter sequence on the 5'-end thereof), the second primer is one type of oligonucleotide selected from SEQ ID NO: 19 to 22, and the intercalating fluorescent dye-labeled oligonucleotide probe is an oligonucleotide consisting of SEQ ID NO: 28 or 29;
(iii) the cleaving nucleotide is an oligonucleotide consisting of SEQ ID NO: 7, the first primer is an oligonucleotide consisting of SEQ ID NO: 14 (and furthermore, the first primer has a promoter sequence on the 5'-end thereof), the second primer is one type of oligonucleotide selected from SEQ ID NO: 20 to 25, and the intercalating fluorescent dye-labeled oligonucleotide probe is one type of oligonucleotide selected from SEQ ID NO: 29 to 31;
(iv) the cleaving nucleotide is an oligonucleotide consisting of SEQ ID NO: 8, the first primer is an oligonucleotide consisting of SEQ ID NO: 15 (and furthermore, the first primer has a promoter sequence on the 5'-end thereof), the second primer is one type of oligonucleotide selected from SEQ ID NO: 20 to 25, and the intercalating fluorescent dye-labeled oligonucleotide probe is one type of oligonucleotide selected from SEQ ID NO: 29 to 31;
(v) the cleaving nucleotide is an oligonucleotide consisting of SEQ ID NO: 9, the first primer is an oligonucleotide consisting of SEQ ID NO: 16 (and furthermore, the first primer has a promoter sequence on the 5'-end thereof), the second primer is one type of oligonucleotide selected from SEQ ID NO: 20 to 25, and the intercalating fluorescent dye-labeled oligonucleotide probe is one type of oligonucleotide selected from SEQ ID NO: 29 to 31;
(vi) the cleaving nucleotide is an oligonucleotide consisting of SEQ ID NO: 10, the first primer is an oligonucleotide consisting of SEQ ID NO: 17 (and furthermore, the first primer has a promoter sequence on the 5'-end thereof), the second primer is one type of oligonucleotide selected from SEQ ID NO: 23 to 25, and the intercalating fluorescent dye-labeled oligonucleotide probe is an oligonucleotide consisting of SEQ ID NO: 30 or 31; and,
(vii) the cleaving nucleotide is an oligonucleotide consisting of SEQ ID NO: 11, the first primer is an oligonucleotide consisting of SEQ ID NO: 18 (and furthermore, the first primer has a promoter sequence on the 5'-end thereof), the second primer is one type of oligonucleotide selected from SEQ ID NO: 23 to 25, and the intercalating fluorescent dye-labeled oligonucleotide probe is an oligonucleotide consisting of SEQ ID NO: 30 or 31.

Each enzyme is required in the method of measuring survivin mRNA of the present invention (including an enzyme having RNA-dependent DNA polymerase activity using single-stranded RNA as a template (reverse transcriptase), an enzyme having RNaseH activity, an enzyme having DNA-dependent DNA polymerase activity that uses single-stranded DNA as a template, and an enzyme having RNA polymerase activity). An enzyme having a combination of several activities may be used, or a plurality of enzymes having each activity may be used for each of the enzymes. In addition, not only may an enzyme having RNA polymerase activity be added to a reverse transcriptase having three kinds of activities of RNA-dependent DNA polymerase activity which uses single-stranded RNA as a template, RNase H activity and DNA-dependent DNA polymerase activity which uses single-stranded DNA as a template, but also an enzyme having RNase H activity may be added as necessary. AMV reverse transcriptase commonly used in molecular biology experiments and the like, MMLV reverse transcriptase, HIV reverse transcriptase or derivatives thereof are preferable for the aforementioned reverse transcriptase, while AMV reverse transcriptase and derivatives thereof are the most preferable. In addition, examples of the aforementioned enzymes having RNA polymerase activity include bacteriophage-derived T7 RNA polymerase commonly used in molecular biology experiments and the like, T3 RNA polymerase, SP6 RNA polymerase and derivatives thereof.

In one aspect of the present invention, the cleaving oligonucleotide is added to survivin mRNA in a sample, and the RNA is cleaved at a 5'-end site of the specific base sequence by RNase H activity of the aforementioned reverse transcriptase. When a reverse transcription reaction is carried out with the reverse transcriptase in the presence of the first primer and the second primer by using the cleaved RNA as a template, the second primer binds to the specific base sequence within the survivin mRNA, and cDNA synthesis is carried out by RNA-dependent DNA polymerase activity of the reverse transcriptase. The first primer binds to the cDNA as a result of the RNA moiety of the resulting double-stranded RNA-DNA being decomposed and dissociated by the RNase H activity of the reverse transcriptase. Continuing, double-stranded DNA derived from the specific base sequence and having a promoter sequence on the 5'-end thereof is formed by the DNA-dependent DNA polymerase activity of the reverse transcriptase. This double-stranded DNA contains the specific base sequence downstream from the promoter sequence, and produces an RNA transcription product derived from the specific base sequence by the RNA polymerase. The RNA transcription product serves as a template for the double-stranded DNA synthesis by the first and second primers, and the RNA transcription product is amplified as a result of a series of reactions proceeding in the manner of a chain reaction.

In order to allow this chain reaction to proceed, it goes without saying that each of the enzymes at least contains as essential known elements a buffer (such as Tris), a magnesium salt, a potassium salt, a nucleoside triphosphate and a ribonucleoside triphosphate. In addition, dimethylsulfoxide (DMSO), dithiothreitol (DTT), bovine serum albumin (BSA) and a sugar and the like may also be added as additives for adjusting reaction efficiency.

For example, in the case of using AMV reverse transcriptase and T7 RNA polymerase, the reaction temperature is preferably set within the range of 35 to 65°C and set particularly preferably within the range of 40 to 50°C. The reaction temperature can be set to an arbitrary temperature at which the RNA amplification step proceeds at a constant temperature and the reverse transcriptase and RNA polymerase demonstrate activity.

The amplified RNA transcription product can be measured according to a known nucleic acid measurement method. Examples of such methods include a method that uses electrophoresis or liquid chromatography, and a hybridization method that uses a nucleic acid probe labeled with a detectable label. However, the procedures of these methods have a large number of steps, and there is a considerable risk of dispersion of the amplification product into the atmosphere causing secondary contamination since the amplification product is analyzed by removing from the reaction system. In order to overcome these shortcomings, it is preferable to use an oligonucleotide probe designed so that fluorescent properties change due to complementary binding with a target nucleic acid. Although a known probe that uses FRET in the manner of a molecular beacon can be used for the oligonucleotide probe, in consideration of ease of designing the probe and ease of probe synthesis, a more preferable method consists of carrying out the nucleic acid amplification step in the presence of an oligonucleotide probe, which is labeled with an intercalating fluorescent dye and designed so that when it forms a complementary double strand with the target nucleic acid, fluorescent properties thereof change as a result of the intercalating fluorescent dye moiety intercalating into the complementary double-strand moiety followed by measuring the change in fluorescent properties (see Patent Document 4 and Non-Patent Document 4).

Although there are no particular limitations thereon, examples of the intercalating fluorescent dye that can be used include commonly used oxazole yellow, thiazole orange, ethidium bromide and derivatives thereof. An example of the change in fluorescent properties described above is a change in fluorescence intensity. In the case of oxazole yellow, for example, fluorescence at 510 nm (excitation wavelength: 490 nm) is known to increase remarkably following intercalation of double-stranded DNA. The aforementioned intercalating fluorescent dye-labeled oligonucleotide probe has a structure in which an intercalating fluorescent dye is bound to an end, phosphate diester moiety or nucleotide moiety in an oligonucleotide complementary to a target RNA of the RNA transcription product through a suitable linker, and the hydroxyl group on the 3'-end is suitably modified for the purpose of preventing elongation from the hydroxyl group on the 3'-end (see Patent Document 4 and Non-Patent Document 4).

Labeling of the intercalating fluorescent dye to an oligonucleotide can be carried out by binding the intercalating fluorescent dye by introducing a functional group into an oligonucleotide using a known method (see Patent Document 4 and Non-Patent Document 4). In addition, commercially available Label-ON Reagents (Clontech) and the like can also be used to introduce the functional group.

In one aspect of the present invention, method is provided consisting of adding an amplification reagent, at least containing a first primer having a T7 promoter sequence (SEQ ID NO: 34) on the 5'-end thereof, a second primer, an intercalating fluorescent dye-labeled oligonucleotide probe, a cleaving oligonucleotide, AMV reverse transcriptase, T7 RNA polymerase, buffer, magnesium salt, potassium salt, nucleoside triphosphate, ribonucleoside triphosphate and dimethylsulfoxide (DMSO), to a sample, and then reacting at a constant reaction temperature of 35 to 65°C (and preferably 40 to 50°C) simultaneous to measuring fluorescence intensity of the reaction solution over time.

In this aspect, since fluorescence intensity is measured over time, measurement can be completed at an arbitrary time at which a significant increase in fluorescence has been observed, and can usually be completed within 20 minutes for both nucleic acid amplification and measurement.

In one aspect of the present invention, an amount of the specific base sequence (number of copies of a target RNA) present in a sample can be calculated by measuring survivin mRNA in the sample and comparing resulting information on fluorescence intensity with information on fluorescence intensity when survivin mRNA was measured at a known concentration. Although a sandwich assay can be applied to detect the specific base sequence by using a immobilized and labeled probe capable of complementarily binding to the specific base sequence in a reaction solution in which the aforementioned reaction was carried out for a fixed period of time, as was previously described, a method that uses an intercalating fluorescent dye-labeled oligonucleotide probe that specifically binds to the specific base sequence is preferable. In addition, since this probe does not impair the aforementioned RNA amplification reaction, a method consisting of carrying out nucleic acid amplification of the specific nucleic acid sequence in the presence of this probe and monitoring the specific nucleic acid sequence amplification status is particularly preferable. Furthermore, in the case of carrying out amplification of a specific nucleic acid sequence in the presence of an intercalating fluorescent dye-labeled oligonucleotide probe, it is preferable to add glycolic acid or biotin to the 3'-end thereof, for example, so as to prevent the probe moiety from functioning as a elongation reaction primer. By then measuring a fluorescence signal emitted as a result of the intercalating fluorescent dye-labeled oligonucleotide probe binding to the specific nucleic acid sequence during the amplification reaction with a fluorescence detector, and comparing information obtained a profile thereof (for example, reaction time required for intensity of fluorescence emitted by the fluorescent dye to reach a fixed intensity) with information obtained from a profile relating to a known amount of standard RNA, the presence or absence of the specific nucleic acid sequence can be confirmed, or the amount of the specific nucleic acid sequence present in the sample (number of copies of a target RNA) can be estimated from the amount of specific nucleic acid sequence (number of RNA copies) that has been amplified.

In addition, it is noteworthy that all samples contained in the measurement reagent can be sealed within a single container. Namely, by simply carrying out a procedure in which a fixed amount of a sample is dispensed into a single container, survivin mRNA can then be subsequently measured automatically. As long as at least a portion of the container is composed of a transparent material so as to enable a signal emitted by the fluorescent dye to be measured from the outside, for example, a container that can be sealed after dispensing the sample is particularly preferable in terms of preventing contamination.

Since the RNA amplification and measurement method of the previous aspects can be carried out in a single step and at a constant temperature, it can be said to be simpler and more suitable to automation than RT-PCR. The present invention makes it possible for the first time to measure survivin mRNA with high specificity, high sensitivity, rapidly, easily and at a constant temperature and in a single step.

In the present invention, an initial amount of RNA can be determined both easily and rapidly by analyzing the course of increases in fluorescence intensity in a step in which double-stranded DNA having a DNA-dependent RNA polymerase promoter region on the 5'-end thereof is synthesized based on a target RNA in a sample (mRNA of survivin gene), a large amount of single-stranded RNA is formed by using this DNA as a template to dramatically increase the amount of the single-stranded RNA formed, and an increase in fluorescence is measured by allowing an intercalating fluorescent dye-labeled oligonucleotide probe to complementarily bind with the single-stranded RNA formed. The combined nucleic acid amplification and measurement time of the survivin mRNA measurement method of the present invention is 30 minutes or less, and this is faster than measurement using a conventional method such as RT-PCR (normally 2 hours or more), NASBA (90 minutes or more) or TMA (90 minutes or more).

Moreover, by providing an oligonucleotide for amplifying and detecting mRNA of survivin gene in a single step, namely by providing an oligonucleotide for amplifying survivin mRNA and an oligonucleotide for detecting survivin mRNA, a simple, fast and highly sensitive method for measuring cells expressing survivin mRNA, and a measurement reagent for use in the fields of biochemistry, molecular biology and medicine, can be provided by using this oligonucleotide.

### Examples

Although the following provides a more detailed explanation of the present invention through examples thereof, these examples are only intended to be exemplary, and the present invention is not limited by these examples.

### Example 1 Preparation of Reference RNA,

Survivin RNA used in the following examples (to be referred to as standard RNA) was prepared using the methods indicated (1) to (2) below.
(1) Double-stranded DNA was cloned consisting of nucleotides from positions 68 to 1328 (1261 nucleotides) of a survivin base sequence registered with GenBank (GenBank Accession No. NM_001012271, 2724 nucleotides).
(2) In vitro transcription was carried out using the double-stranded DNA prepared in (1) as a template, and RNA was prepared after completely digesting the double-stranded DNA by treating with DNase I and purifying. The RNA was quantified by measuring absorbance at 260 nm.

### Example 2 Preparation of Intercalating Fluorescent Dye-Labeled Oligonucleotide Probe

An oligonucleotide probe that was labeled with an intercalating fluorescent dye was prepared. An amino group was respectively introduced using Label-ON Reagents (Clontech) at the location of the 11th C from the 5'-end of the sequence described in SEQ ID NO: 28, the 11th G from the 5'-end of the sequence described in SEQ ID NO: 29, the 11th A from the 5'-end of the sequence described in SEQ ID NO: 30, the 11th A from the 5'-end of the sequence described in SEQ ID NO: 31, the 11th C from the 5'-end of the sequence described in SEQ ID NO: 32, and the 11th A from the 5'-end of the sequence described in SEQ ID NO: 33, followed by further modifying the 3'-end with biotin. Oxazole yellow serving as intercalating fluorescent dye was labeled to each amino group to prepare oxazole yellow-labeled oligonucleotide probes (SEQ ID NO: 28 to 33) (see Ishiguro, T. et al., Nucleic Acids Res., 24, 4992-4997 (1996) (Non-Patent Document 5)).

### Example 3 Measurement of Survivin RNA (Part 1)

Standard RNA was measured according to the method indicated in (1) to (4) using the first primers, second primers, intercalating fluorescent dye-labeled oligonucleotide probes (to be referred to as "INAF probes") and cleaving oligonucleotides indicated in the combinations of [1] to [38] shown in Table 1. Furthermore, in Table 1, SEQ ID NO: 12 and 13 constitute partial sequences of SEQ ID NO: 1, SEQ ID NO: 14 to 18 constitute partial sequences of SEQ ID NO: 2, SEQ ID NO: 19 to 22 constitute partial sequences of SEQ ID NO: 3, and SEQ ID NO: 20 to 25 constitute partial sequences of SEQ ID NO: 4.

**Table 1**

| Oligonucleotide combination | Oligonucleotides Used (SEQ ID NO) | | | |
|---|---|---|---|---|
| | Cleaving oligonucleotide | First primer | Second primer | INAF probe |
| [1] | 5 | 12 | 19 | 28 |
| [2] | 5 | 12 | 20 | 29 |
| [3] | 5 | 12 | 21 | 29 |
| [4] | 5 | 12 | 22 | 29 |
| [5] | 6 | 13 | 19 | 28 |
| [6] | 6 | 13 | 20 | 28 |
| [7] | 6 | 13 | 20 | 29 |
| [8] | 6 | 13 | 21 | 29 |
| [9] | 6 | 13 | 22 | 28 |
| [10] | 6 | 13 | 22 | 29 |
| [11] | 7 | 14 | 23 | 29 |
| [12] | 7 | 14 | 24 | 30 |
| [13] | 8 | 15 | 20 | 29 |
| [14] | 8 | 15 | 21 | 29 |
| [15] | 8 | 15 | 22 | 29 |
| [16] | 8 | 15 | 23 | 29 |
| [17] | 8 | 15 | 23 | 30 |
| [18] | 8 | 15 | 23 | 31 |
| [19] | 8 | 15 | 24 | 30 |
| [20] | 8 | 15 | 25 | 29 |
| [21] | 9 | 16 | 23 | 30 |
| [22] | 9 | 16 | 24 | 31 |
| [23] | 10 | 17 | 23 | 30 |
| [24] | 10 | 17 | 24 | 31 |
| [25] | 10 | 17 | 25 | 30 |
| [26] | 11 | 18 | 23 | 31 |
| [27] | 11 | 18 | 24 | 30 |
| [28] | 11 | 18 | 25 | 31 |
| [29] | 5 | 12 | 26 | 28 |
| [30] | 5 | 12 | 26 | 32 |
| [31] | 5 | 12 | 27 | 28 |
| [32] | 5 | 12 | 27 | 32 |
| [33] | 6 | 13 | 26 | 28 |
| [34] | 6 | 13 | 26 | 32 |
| [35] | 6 | 13 | 27 | 28 |
| [36] | 6 | 13 | 27 | 32 |
| [37] | 7 | 14 | 19 | 33 |
| [38] | 11 | 18 | 23 | 30 |

(1) The standard RNA prepared in Example 1 were diluted using RNA diluent (10 mM Tris-HCl buffer (pH 8.0), 0.1 mM EDTA, 0.5 U/µL ribonuclease inhibitor, 5.0 mM DTT) to 10³ copies/5 µL and used as RNA samples.
(2) 20 µL aliquots of the reaction solution having the composition indicated below were dispensed into 0.5 mL volume PCR tubes (Individual PCR Tubes with Dome Cap, SSI) followed by the addition of 5 µL of the RNA samples thereto.
   Reaction Solution Composition (Final concentration after addition of enzyme solution (in 30 µL))
   60 mM Tris-HCl buffer (pH 8.6)
   18 mM magnesium chloride
   100 mM potassium chloride
   1.0 mM DTT
   0.25 mM each dATP, dCTP, dGTP, dTTP
   3.0 mM each ATP, CTP, UTP, GTP
   3.6 mM ITP
   1.0 µM first primer (a T7 promoter sequence (SEQ ID NO: 34) was added to the 5'-end of the primer having the
   base sequence described in each SEQ ID NO)
   1.0 µM second primer
   0.16 µM cleaving oligonucleotide (a hydroxyl group on the 3'-end of the oligonucleotide is modified with an amino group)
   20 nM INAF probe (the probe prepared in Example 2)
   6.0 U ribonuclease inhibitor (Takara Bio)
   13% DMSO
   Distilled water for adjusting volume
(3) The reaction solution was warmed for 5 minutes at 43°C followed by adding 5.0 µL of an enzyme solution having the composition indicated below pre-warmed for 2 minutes at 43°C.
   Enzyme Solution Composition (Final concentration at time of reaction (in 30 µL))
   2.0% Sorbitol
   6.4 U AMV reverse transcriptase (Life Science)
   142 U T7 RNA polymerase (Invitrogen)
   3.6 µg bovine serum albumin (Takara Bio)
   Distilled water for adjusting volume
(4) Continuing, fluorescence intensity was measured (excitation wavelength: 470 nm, fluorescence wavelength: 510 nm) over time for 20 minutes simultaneous to reacting at 43°C using a fluorescence spectrophotometer equipped with a temperature control function capable of measuring the PCR tubes directly.

Designating the time of addition of enzyme as 0 minutes, samples were judged to be positive when the fluorescence intensity ratio of the reaction solution (fluorescence intensity value at a prescribed time ÷ background fluorescence intensity value) exceeded 1.2, and results showing the elapsed times at that time are shown in Table 2 as detection times. Furthermore, N.D. in Table 2 indicates samples for which the fluorescence intensity ratio at 20 minutes after enzyme addition was less than 1.2 (negative result).

**Table 2**

| Oligonucleotide combination | Detection time (min) (10³ copies/test) | Oligonucleotide combination | Detection time (min) (10³ copies/test) |
|---|---|---|---|
| [1] | 13.85 | [20] | 11.81 |
| [2] | 12.16 | [21] | 8.95 |
| [3] | 11.03 | [22] | 10.66 |
| [4] | 10.35 | [23] | 8.99 |
| [5] | 10.37 | [24] | 10.50 |
| [6] | 13.25 | [25] | 8.37 |
| [7] | 10.39 | [25] | 9.39 |
| [8] | 12.32 | [27] | 6.97 |
| [9] | 11.96 | [28] | 14.08 |
| [10] | 9.32 | [29] | N.D. |
| [11] | 15.33 | [30] | N.D. |
| [12] | 11.85 | [31] | N.D. |
| [13] | 11.14 | [32] | N.D. |
| [14] | 9.24 | [33] | N.D. |
| [15] | 13.44 | [34] | N.D. |
| [16] | 10.99 | [35] | N.D. |
| [17] | 8.67 | [36] | N.D. |
| [18] | 9.86 | [37] | N.D. |
| [19] | 8.49 | | |

Among the combinations of Table 1:
(i) in those combinations in which the first primer is consisting of a partial sequence of SEQ ID NO: 1 and the second primer is consisting of a partial sequence of SEQ ID NO: 3 (combinations [1] to [10]), and
(ii) in those combinations in which the first primer is consisting of a partial sequence of SEQ ID NO: 2 and the second primer is consisting of a partial sequence of SEQ ID NO: 4 (combinations [11] to [28]),

10³ copies/5 µL of the standard RNA were detected within 20 minutes for all combinations. On the other hand, those combinations other than those indicated in (i) and (ii) above (combinations [29] to [37]) all demonstrated negative results. Furthermore, fluorescence intensity ratios did not exceed 1.2 even after 30 minutes from the start of the reaction in a control test group (which were measured by adding RNA diluent to the reaction solution instead of standard RNA).

According to these results, it was indicated that by carrying out an RNA amplification reaction using:
(i) combinations in which the first primer is an oligonucleotide consisting of at least 15 contiguous bases in the base sequence listed as SEQ ID NO: 1 and the second primer is an oligonucleotide consisting of at least 15 contiguous bases in the base sequence listed as SEQ ID NO: 3, or
(ii) the first primer is an oligonucleotide consisting of at least 15 contiguous bases in the base sequence listed as SEQ ID NO: 2 and the second primer is an oligonucleotide consisting of at least 15 contiguous bases in the base sequence listed as SEQ ID NO: 4,

survivin RNA is detected more rapidly in comparison with detection of survivin mRNA by the RT-PCR method of the prior art (Non-Patent Documents 1 to 3).

### Example 4 Measurement of Survivin RNA (Part 2)

The relationship between detection time and initial amount of standard RNA was confirmed by measuring various concentrations of standard RNA using combinations of oligonucleotides of the present invention.

### (1) Experiment Method

Measurements were carried out using the same method as Example 3 with the exception of changing the combinations of oligonucleotides and RNA samples to that indicated below.

### Combinations of oligonucleotides:

Combinations [7], [15], [17] and [38] of Table 1 were used.

### RNA samples:

The standard RNA prepared in Example 1 was diluted with the RNA diluent used in part (1) of Example 3 to 3 × 10² copies/5 µL, 3 × 10³ copies/5 µL, 3 × 10⁴ copies/5 µL or 3 × 10⁵ copies/5 µL in combination [7], and diluted to 10² copies/5 µL, 10³ copies/5 µL, 10⁴ copies/5 µL or 10⁵ copies/5 µL in the other combinations.

### (2) Experiment Results

Designating the time of addition of enzyme as 0 minutes, samples were judged to be positive when the fluorescence intensity ratio of the reaction solution (fluorescence intensity value at a prescribed time ÷ background fluorescence intensity value) exceeded 1.2, and results showing the elapsed times at that time are shown in Tables 3 and 4 as detection times, while the results of plotting a calibration curve based on the results of Tables 3 and 4 are shown in FIG. 1.

**Table 3**

| Oligonucleotide combinations | Detection time (min) at each standard RNA concentration (copies/test) | | | |
|---|---|---|---|---|
| | 3×10² copies | 3×10³ copies | 3×10⁴ copies | 3×10⁵ copies |
| [7] | 11.21 | 9.52 | 8.40 | 7.54 |

**Table 4**

| Oligonucleotide combinations | Detection time (min) at each standard RNA concentration (copies/test) | | | |
|---|---|---|---|---|
| | 10² copies | 10³ copies | 10⁴ copies | 1.0⁵ copies |
| [15] | 10.54 | 9.62 | 8.80 | 8.16 |
| [17] | 10.23 | 9.26 | 8.29 | 7.44 |
| [38] | 10.14 | 8.66 | 7.31 | 6.22 |

All concentrations of the measured standard RNA were detected within 15 minutes after enzyme addition for all combinations of oligonucleotides examined in this experiment. In addition, when detection time was plotted on the vertical axis and the initial amount of standard RNA (represented as a log value of the number of copies) was plotted on the horizontal axis, detection times were dependent on the initial amount of standard RNA starting in a low copy region of 3 × 10² copies or less, and the calibration curve was able to be approximated to a linear first order curve. Namely, it was indicated that by measuring mRNA according to the method of the present invention for an unknown sample and applying the resulting detection time to the calibration curve shown in FIG. 1, the amount of survivin mRNA contained in the unknown sample can be estimated.

### Example 5 Measurement of Survivin RNA (Part 3)

Survivin mRNA present in an extract was quantified by measuring a commercially available human bladder tumor extract using combinations of oligonucleotides of the present invention.

### (1) Experiment Method

Measurements were carried out using the same method as Example 3 with the exception of changing the combinations of oligonucleotides and RNA samples to that indicated below.

### Combinations of oligonucleotides:

Combinations [7], [15], [17] and [38] of Table 1 were used.

### RNA samples:

A commercially available human bladder tumor extract (FirstChoice Tumor RNA: Human Bladder Tumor RNA (Ambion)) was diluted with the RNA diluent used in part (1) of Example 3 to concentrations from 0.005 ng/5 µL to 50 ng/5 µL.

### (2) Experiment Results

Designating the time of addition of enzyme as 0 minutes, samples were judged to be positive when the fluorescence intensity ratio of the reaction solution (fluorescence intensity value at a prescribed time ÷ background fluorescence intensity value) exceeded 1.2, and results showing the elapsed times at that time are shown in Table 5 as detection times, while the results of calculating the amount of survivin mRNA contained in each RNA sample based on the detection times of Table 5 and the calibration curve obtained in Example 4 are shown in Table 6. Furthermore, N.D. in Table 5 indicates samples for which the fluorescence intensity ratio at 20 minutes after enzyme addition was less than 1.2 (negative result).

**Table 5**

| Oligonucleotide combinations | Detection time (min) for each extract RNA sample (ng/test) | | | | |
|---|---|---|---|---|---|
| | 0.005 ng | 0.05 ng | 0.5 ng | 5 ng | 50 ng |
| [7] | N.D. | 16.20 | 12.09 | 10.37 | 8.73 |
| [15] | 14.81 | 13.21 | 10.57 | 9.37 | 8.32 |
| [17] | N.D. | 10.91 | 9.94 | 8.46 | 7.70 |
| [38] | N.D. | N.D. | 8.95 | 8.23 | 6.86 |

**Table 6**

| Oligonucleotide combinations | Amount of survivin mRNA (copies) in each extract RNA sample (ng/test) | | | | |
|---|---|---|---|---|---|
| | 0.005 ng | 0.05 ng | 0.5 ng | 5 ng | 50 ng |
| [7] | N.D. | 0 | 35 | 846 | 18818 |
| [15] | 0 | 0 | 76 | 2437 | 50819 |
| [27] | N.D. | 18 | 193 | 7403 | 48204 |
| [38] | N.D. | N.D. | 689 | 2441 | 27070 |

The amounts of survivin mRNA in each of the RNA samples determined from the calibration curve were nearly proportional to the total amount of RNA used, thereby demonstrating that the survivin RNA measurement method of the present invention is concentration-dependent. Furthermore, combination [17] resulted in detection of the lowest concentration of RNA sample among the oligonucleotide combinations examined in this experiment.

### Example 6 Nucleotide Sequence Analysis of Survivin RNA Amplification Product

The base sequences were analyzed for the double-stranded DNA contained in the samples obtained following the nucleic acid amplification reaction in Example 5. As a result of analyzing the base sequences, corresponding base sequences yielded by survivin mRNA amplification were confirmed for samples amplified using each of the combinations of oligonucleotides.

In other words, this indicates that survivin RNA is measured in the present invention, and not other non-specific RNA. Although contaminating RNA was included in addition to survivin RNA in the commercially available extract used in Example 4, the measurement method of the present invention only measured survivin mRNA, and can be said to be extremely specific.

On the basis of the above, the method of measuring survivin mRNA of the present invention enables survivin mRNA contained in an unknown sample to be measured specifically, rapidly and with high sensitivity, and was also indicated to enable quantification by using a calibration curve.

### INDUSTRIAL APPLICABILITY

According to the present invention, survivin RNA can be measured specifically, rapidly and with high sensitivity in a single step under comparatively low and constant temperature conditions (40 to 50°C, and preferably 43°C).

## Claims

1. A method of measuring survivin mRNA in a sample, consisting of the following steps that uses a first primer having a sequence homologous with a portion of a specific base sequence in survivin mRNA, and a second primer having a sequence complementary with a portion of the specific base sequence, and one of either the first primer or the second primer is a primer to which has been added to the 5'-end thereof an RNA polymerase promoter sequence:
(1) a step for synthesizing a cDNA complementary to said specific base sequence by use of an enzyme having RNA-dependent DNA polymerase activity which uses RNA as a template;
(2) a step for decomposing the RNA-DNA double-stranded RNA obtained in the reaction of (1) above by use of an enzyme having ribonuclease H (RNase H) activity (formation of single-stranded DNA);
(3) a step for forming double-stranded DNA having a promoter sequence capable of transcribing the specific base sequence or RNA having a sequence complementary to the specific base sequence by use of an enzyme having DNA-dependent DNA polymerase activity with using the single-stranded DNA as template;
(4) a step for forming an RNA transcription product by use of an enzyme having RNA polymerase activity with using the double-stranded DNA as template;
(5) a step for forming an RNA transcription product in a chain reaction by allowing the RNA transcription product to serve as a template of cDNA synthesis in the reaction of (1); and,
(6) a step for measuring the amount of the RNA transcription product;
wherein the first and second primers are either of the oligonucleotides indicated below:
(i) the first primer is one type of oligonucleotide selected from SEQ ID NO: 12 or 13, and the second primer is one type of oligonucleotide selected from any of SEQ ID NO: 19 to 22; or,
(ii) the first primer is one type of oligonucleotide selected from any of SEQ ID NO: 14 to 18, and the second primer is one type of oligonucleotide selected from any of SEQ ID NO: 20 to 25; and
wherein said method is carried out under constant temperature conditions from 40°C to 50°C.

2. The method of measuring survivin mRNA according to claim 1, **characterized in that** the step described in (6) above (the step for measuring an amount of RNA transcription product) is carried out by measuring a change in fluorescent properties in the presence of a fluorescent dye-labeled oligonucleotide probe designed to change in its fluorescent properties when it forms a complementary double-strand with the target RNA.

3. The method of measuring survivin mRNA according to claim 2, **characterized in that** the fluorescent dye-labeled oligonucleotide probe is an intercalating fluorescent dye-labeled oligonucleotide probe in which an intercalating fluorescent dye is bound through a linker.

4. The method of measuring survivin mRNA according to any of claims 1 to 3, **characterized in that** prior to the step described in (1) above (the step for synthesizing cDNA complementary to the specific base sequence by use of an enzyme having RNA-dependent DNA polymerase activity which uses with RNA as a template), a step is carried out for cleaving the RNA at the 5'-end site of the specific base sequence by use of a specific base sequence in survivin mRNA as a template, and using:
(i) a cleaving oligonucleotide having a region that overlaps with the 5'-end site of a region homologous to the first primer in the specific base sequence, and a sequence complementary to an adjacent region on the 5' side from the site, and
(ii) an enzyme having ribonuclease H (RNase H) activity.

5. An oligonucleotide combination for specifically amplifying or detecting survivin mRNA, **characterized by** containing a first primer and a second primer, wherein the first and second primers are either of the oligonucleotides indicates below :
(i) the first primer is one type of oligonucleotide selected from SEQ ID NO: 12 or 13, and the second primer is one type of oligonucleotide selected from any of SEQ ID NO: 19 to 22; or,
(ii) the first primer is one type of oligonucleotide selected from any of SEQ ID NO: 14 to 18, and the second primer is one type of oligonucleotide selected from any of SEQ ID NO: 20 to 25.

6. A reagent for measuring survivin mRNA, **characterized by** containing at least the oligonucleotide combination of claim 5.

## Patentansprüche

1. Verfahren zum Messen der Menge an Survivin mRNA in einer Probe, bestehend aus den folgenden Schritten, in welchen ein erster Primer verwendet wird, der eine Sequenz aufweist, die homolog zu einem Teil einer spezifischen Basensequenz in der Survivin mRNA ist, sowie einen zweiten Primer, mit einer Sequenz, die zu einem Teil der spezifischen verwendet Basensequenz komplementär ist, wobei entweder der erste Primer oder der zweite Primer ein Primer ist, der dem 5'-Ende einer RNA-Polymerase-Promotorsequenz hinzugefügt worden ist:
(1) einen Schritt zum Synthetisieren einer cDNA, die komplementär zu besagter spezifischer Basensequenz ist, unter Verwendung eines Enzyms, welches RNA-abhängige DNA-Polymerase-Aktivität aufweist, und welches RNA als Templat verwendet;
(2) einen Schritt zum Zerlegen der RNA-DNA-Doppelstrang-RNA, welche in der Reaktion von (1) oben unter Verwendung eines Enzyms mit Ribonuclease H (RNase H) Aktivität erhalten wurde (Bildung von Einzelstrang-DNA);
(3) einen Schritt zur Bildung doppelsträngiger DNA mit einer Promotorsequenz, welche zur Transkription der spezifischen Basensequenz geeignet ist, oder einer RNA mit einer Sequenz, die komplementär zur spezifischen Basensequenz ist, unter Verwendung eines Enzyms mit DNA-abhängiger DNA-Polymerase-Aktivität unter Verwendung der einzelsträngigen DNA als Templat;
(4) einen Schritt zum Bilden eines RNA-Transkriptionsprodukts unter Verwendung eines Enzyms mit RNA-Polymerase-Aktivität, unter Verwendung der doppelsträngigen DNA als Templat;
(5) einen Schritt zum Bilden eines RNA-Transkriptionsprodukt in einer Kettenreaktion, indem dem RNA-Transkriptionsprodukt erlaubt wird, als Templat für die cDNA-Synthese in der Reaktion (1) zu dienen; und
(6) einen Schritt zum Messen der Menge des RNA-Transkriptionsprodukts; wobei die ersten und zweiten Primer jeweils eines der folgenden Oligonukleotide sind:
(i) der erste Primer ist eine Art von Oligonukleotid ausgewählt aus SEQ ID NO: 12 oder 13, und der zweite Primer ist eine Art von Oligonukleotid ausgewählt aus jedem der SEQ ID NO: 19 bis 22; oder
(ii) der erste Primer ist eine Art von Oligonukleotid ausgewählt aus SEQ ID NO: 14 bis 18, und der zweite Primer ist eine Art von Oligonukleotid ausgewählt aus jedem der SEQ ID NO: 20 bis 25; und
wobei die besagte Methode unter konstanten Temperaturbedingungen von 40°C bis 50°C ausgeführt wird.

2. Das Verfahren zum Messen der Survivin mRNA nach Anspruch 1, **dadurch gekennzeichnet, dass** der oben in (6) beschriebene Schritt (Schritt zum Messen der Menge an RNA Transkriptionsprodukt) so ausgeführt wird, dass einer Änderung der Fluoreszenzeigenschaften gemessen wird, und zwar in Gegenwart einer mit einem fluoreszierenden Farbstoff markierten Oligonucleotid-Sonde, welche so eingerichtet ist, dass sie ihre Fluoreszenzeigenschaften ändert, wenn diese einen komplementäre Doppelstrang mit der Ziel-RNA bildet.

3. Das Verfahren zum Messen der Survivin mRNA nach Anspruch 2, **dadurch gekennzeichnet, dass** die mit einem fluoreszierenden Farbstoff-markierte Oligonukleotid-Sonde eine mit einem interkalierenden fluoreszierenden Farbstoff markierte Oligonucleotid-Sonde ist, in welcher ein interkalierender fluoreszierender Farbstoff über einen Linker gebunden ist.

4. Das Verfahren zum Messen der Survivin mRNA nach einem der Ansprüche 1 bis 3, **dadurch gekennzeichnet, dass** vor dem oben in (1) beschriebenen Schritt (dem Schritt zum Synthetisieren einer cDNA, die komplementär zur spezifischen Basensequenz ist, unter Verwendung eines Enzyms, welches RNA-abhängige DNA-Polymerase-Aktivität aufweist, und welches RNA als Templat verwendet) ein Schritt zum Spalten der RNA an der 5'-Endestelle des spezifischen Basensequenz ausgeführt wird, unter Verwendung einer spezifischen Basensequenz in der Survivin mRNA als Templat eingesetzt wird, und unter Verwendung von:
(i) einem spaltenden Oligonukleotid mit einer Region, welche mit der 5'-Endstelle einer Region überlappt, die zum der ersten spezifischen Primer homolog im Hinblick auf die Basensequenz ist, und mit einer Sequenz, die zu einer benachbarten Region auf der 5'-Seite, von der Seite, komplementär ist, und
(ii) einem Enzym mit Ribonuklease H (RNase H)-Aktivität.

5. Eine Oligonukleotid-Kombination zum spezifischen Verstärken oder Detektieren von Survivin mRNA, **dadurch gekennzeichnet, dass** diese einen ersten und einen zweiten Primer enthält, wobei die ersten und zweiten Primer jeweils eines der folgenden Oligonukleotide sind:
(i) der erste Primer ist eine Art von Oligonukleotid ausgewählt aus SEQ ID NO: 12 oder 13, und der zweite Primer ist eine Art von Oligonukleotid ausgewählt aus jedem der SEQ ID NO: 19 bis 22; oder
(ii) der erste Primer ist eine Art von Oligonukleotid ausgewählt aus SEQ ID NO: 14 bis 18, und der zweite Primer ist eine Art von Oligonukleotid ausgewählt aus jedem der SEQ ID NO: 20 bis 25.

6. Ein Reagenz zur Messung von Survivin mRNA, **dadurch gekennzeichnet, dass** es mindestens eine der Oligonukleotid-Kombinationen gemäß Anspruch 5 enthält.

## Revendications

1. Procédé de mesure de l'ARNm de survivine dans un échantillon, constitué des étapes suivantes qui utilise une première amorce ayant une séquence homologue à une partie d'une séquence de bases spécifique dans l'ARNm de survivine, et une deuxième amorce ayant une séquence complémentaire à une partie de la séquence de bases spécifique, et l'une ou l'autre de la première amorce ou de la deuxième amorce est une amorce à laquelle a été ajoutée, à l'extrémité 5' de celle-ci, une séquence promotrice d'ARN polymérase :
(1) une étape consistant à synthétiser un ADNc complémentaire à ladite séquence de bases spécifique par l'utilisation d'une enzyme ayant une activité ADN polymérase dépendant de l'ARN qui utilise l'ARN comme matrice ;
(2) une étape consistant à décomposer l'ARN double brin de l'ARN-ADN obtenu dans la réaction de (1) ci-dessus par l'utilisation d'une enzyme ayant une activité ribonucléase H (RNase H) (formation d'ADN simple brin) ;
(3) une étape consistant à former un ADN double brin ayant une séquence promotrice capable de transcrire la séquence de bases spécifique ou l'ARN ayant une séquence complémentaire à la séquence de bases spécifique par l'utilisation d'une enzyme ayant une activité ADN polymérase dépendant de l'ADN en utilisant l'ADN simple brin comme matrice ;
(4) une étape consistant à former un produit de transcription d'ARN par l'utilisation d'une enzyme ayant une activité ARN polymérase en utilisant l'ADN double brin comme matrice ;
(5) une étape consistant à former un produit de transcription d'ARN dans une réaction en chaîne en permettant au produit de transcription d'ARN de servir de matrice de synthèse d'ADNc dans la réaction de (1) ; et,
(6) une étape consistant à mesurer la quantité du produit de transcription d'ARN ;
dans lequel les première et deuxième amorces sont l'un ou l'autre des oligonucléotides indiqués ci-dessous :
(i) la première amorce est un type d'oligonucléotide choisi parmi la séquence SEQ ID NO : 12 ou 13, et la deuxième amorce est un type d'oligonucléotide choisi parmi l'une des séquences SEQ ID NO : 19 à 22 ; ou,
(ii) la première amorce est un type d'oligonucléotide choisi parmi l'une des séquences SEQ ID NO : 14 à 18, et la deuxième amorce est un type d'oligonucléotide choisi parmi l'une des séquences SEQ ID NO : 20 à 25 ; et
dans lequel ledit procédé est réalisé dans des conditions de température constante allant de 40°C à 50°C.

2. Procédé de mesure de l'ARNm de survivine selon la revendication 1, **caractérisé en ce que** l'étape décrite dans (6) ci-dessus (l'étape consistant à mesurer une quantité de produit de transcription d'ARN) est réalisée en mesurant un changement de propriétés de fluorescence en présence d'une sonde oligonucléotidique marquée par un colorant fluorescent conçue pour changer ses propriétés de fluorescence lorsqu'elle forme un double brin complémentaire à l'ARN cible.

3. Procédé de mesure de l'ARNm de survivine selon la revendication 2, **caractérisé en ce que** la sonde oligonucléotidique marquée par un colorant fluorescent est une sonde oligonucléotidique marquée par un colorant fluorescent intercalant dans laquelle un colorant fluorescent intercalant est lié par un lieur.

4. Procédé de mesure de l'ARNm de survivine selon l'une des revendications 1 à 3, **caractérisé en ce que**, avant l'étape décrite dans (1) ci-dessus (l'étape consistant à synthétiser l'ADNc complémentaire à la séquence de bases spécifique par l'utilisation d'une enzyme ayant une activité ADN polymérase dépendant de l'ARN qui utilise l'ARN comme matrice), une étape est réalisée pour cliver l'ARN au niveau du site d'extrémité 5' de la séquence de bases spécifique par l'utilisation d'une séquence de bases spécifique dans l'ARNm de survivine comme matrice, et en utilisant :
(i) un oligonucléotide de clivage ayant une région qui chevauche le site d'extrémité 5' d'une région homologue à la première amorce dans la séquence de bases spécifique, et une séquence complémentaire à une région adjacente sur le côté 5' du site, et
(ii) une enzyme ayant une activité ribonucléase H (RNase H).

5. Combinaison d'oligonucléotides destinée à amplifier ou à détecter de manière spécifique l'ARNm de survivine, **caractérisée en ce qu'**elle contient une première amorce et une deuxième amorce, où les première et deuxième amorces sont l'un ou l'autre des oligonucléotides indiqués ci-dessous :
(i) la première amorce est un type d'oligonucléotide choisi parmi la séquence SEQ ID NO : 12 ou 13, et la deuxième amorce est un type d'oligonucléotide choisi parmi l'une des séquences SEQ ID NO : 19 à 22 ; ou,
(ii) la première amorce est un type d'oligonucléotide choisi parmi l'une des séquences SEQ ID NO : 14 à 18, et la deuxième amorce est un type d'oligonucléotide choisi parmi l'une des séquences SEQ ID NO : 20 à 25.

6. Réactif de mesure de l'ARNm de survivine, **caractérisé en ce qu'**il contient au moins la combinaison d'oligonucléotides de la revendication 5.
